# EUROPEAN PATENT APPLICATION

(11) **EP 1 903 034 A1**
(43) Date of publication of application: **26.03.2008**
(21) Application number: 06019545.0
(22) Date of filing: 19.09.2006
(51) Int. Cl.: C07D 211/00, C07H 17/02

(54) **Iminosugar glycoconjugates**

(71) Applicant: Technische Universität Graz, 8010 Graz (AT); Forschungsholding TU Graz GmbH, 8010 Graz (AT)
(72) Inventor: Stütz, Arnold, 8020 Graz (AT); Steiner, Andreas, 8010 Graz (AT); Wrodnigg, Tanja, 8010 Graz (AT)

(57) **Abstract**

The iminosugar conjugates according to the invention are N-alkylated 1,5-dideoxy-1,5-iminohexitol or 1,5-dideoxy-1,5-iminopentitol derivatives. The iminosugar component can be, for example, D-*gluco*-, L-*ido*-, D-*galacto*-, D*-manno*-, 2-acetamido-2-deoxy-D-*gluco*- or *xylo*-configuration. The N-substituent is a protected L-α-aminoacid derivative, showing L-lysine-like structural features. The linkage between the carbohydrate and the peptide component is not via the usual glycosidic position, but shows structural features of a very stable tertiary amine. Thus the linkage is very stable. These new compounds are synthesised by using catalytic intramolecular reductive amination of dicarbonyl sugars with partially protected amino acids. The process of intramolecular reductive amination itself is carried out using Pearlman's catalyst (Pd(OH)₂/C) and H₂ at ambient pressure and room temperature.

The resulting accessible class of iminosugar conjugate compounds is represented by the general structure shown in Figure 4(c). The alkyl chain length parameter n can be freely chosen from n=0 upwards. Preferably n is between 0 and 10, and more preferably n is 2, 3, or 4. Residue R¹ can be chosen from H, OH, or NHAc, with Ac being Acetyl. R² can be H, OH, or NHAc. R³, R⁴, R⁵, R⁶ can be H or OH. R⁷ and R⁸ can be H, CH2OH CH3, COQH, or COOR with R being Alkyl or Aryl. R⁹ and R¹⁰ can be chosen from H, NH₂, NHR, with R being a protective group, an amino acid, a peptide, or a protein. R¹¹ can be OH, O-Alkyl, O-Aryl, NH₂, N-Alkyl, N-Aryl, amino acid or peptide, connected via an amide bond.

## Description

### Field of technology

The present invention relates to iminosugar conjugates, a method for the production of such iminosugar conjugates, and peptides and proteins modified with such a method, according to the preamble of the independent claims.

### State of the art

### Biological and medical relevance of glycoconjugates

Glycoconjugates, particularly glycoproteins and glycolipids, are of special biological relevance. A major part of all naturally occurring proteins are glycosylated and carry sugar residues on their surface. Among other functions these carbohydrate groups influence the function, the life time, and the transport of a protein in the body. Glycoconjugates are also known to play an important role in the immune system, in cell tissues as well as in defence processes against infectious diseases.

Dissolved glycoproteins are found in blood (e.g. as blood group determinants) as well as in various secretions. Oligosaccharides are also encountered as components of glycoconjugates on the cell surface, where they are involved in various biochemical recognition processes. Due to the complexity regarding their spatial order, oligosaccharides are able to encode for the gigantic amount of data needed for biological recognition processes, such as growth and evolution, immune defense, introduction of bacterial or viral infections, signal transmission, cell-cell communication and cell adhesion in case of inflammations and metastases. During embryonic evolution, glycoproteins are also responsible for the correct location of cells.

Glycolipids are membrane components and occur in all kingdoms of organisms, i.e., bacteria, plants, and animals including man. They comprise a very heterogeneous group of molecules which possess one or more monosaccharide units linked by glycosidic linkage to a hydrophobic moiety such as acylglycerol, ceramide or prenyl phosphate. In general, glycolipids are important factors of membrane stabilization and cell surface rigidization. They also play an important role in a broad variety of biological processes, e.g. in photosynthetic electron transport in plants, in cell-cell communication, in receptor modulation or in signal transducing. Glycoproteins are macromolecules composed of a protein and several carbohydrates linked to it, generally lacking a repeating unit. The carbohydrate part in these biomolecules varies from less than one percent, e.g. in egg albumin, up to 85%, e.g. in mucoproteins. Proteins are glycosylated post-translationally, and the natural distribution of these biologically important glycoconjugates is almost universal. Covalent association of complex oligosaccharides or low molecular weight polysaccharide moieties occurs at a variety of positions in protein chains, resulting in a multiplicity of structures and biological functions. The presence of carbohydrates influences the secondary structure of the protein backbone, protein conformation, protein solubility and immune response, and provides sites crucial to mutual recognition of, and association with, lectins, viruses and other microorganisms, enzymes and cell surface components. Alterations of the glycan structure may result in the introduction of cancerous growth and metastases. Ever increasing awareness of the scope and potential of chemical and biological investigations of this diverse class of natural biopolymers has promoted a surge in research activity, directed towards the isolation, characterization and physiological testing of glycoproteins, and a corresponding development of synthetic technologies to generate glycopeptides of all kinds.

Artificial glycosylation serves as a means of changing the structural properties of a given protein. Even a single carbohydrate appropriately attached to a peptide can influence its secondary structure and solubility. The glycan can protect the peptide backbone from protolytic attack, as a result of which the introduction of carbohydrates biological half-life of a putative peptide drug can be increased. Glycopeptides are also very potent therapeutic agents in the treatment of cancer. Since the malignant phenotype of cells is characterized by a dramatic transformation of the glycosylation machinery, aberrantly glycosylated proteins are amongst the most occurring tumour antigens. Therefore, synthetic tumour-specific structures can be used as vaccines and the elicited antibodies and T cells do cross-react with the cancer cells.

Most biotechnologically applied processes for the expression of proteins are not able to reproduce correctly glycosylated proteins. Artificial post-translational glycosylation, however, is practically impossible. Therefore polyethylene glycol (PEG) residues are attached to active compounds to increase the lifetime and to improve the pharmacokinetics of a medicament. An example is PEGylated interferon. The production of homogeneous, well-defined derivatives, however, is very difficult. In most cases a mixture of adducts structurally similar adducts is obtained, with similar structure but different physiological activity, which complicates the marketing authorisation of such medicaments.

### Biochemical glycosylation

There are two main types of protein glycosylation: N-glycosylation, in which the saccharide is attached to an asparagine residue, and 0-glycosylation, where the saccharide is linked to the side chain of serine or threonine via an O-glycosidic bond. Less common connections are O-glycosidic linkages to tyrosine, hydroxyproline or hydroxylysine. In-vivo glycosylation occurs in the endoplasmatic reticulum (ER) and Golgi compartments of the cell and involves a complex series of reactions catalyzed by membrane-bound glycosyltransferases and glycosidases.

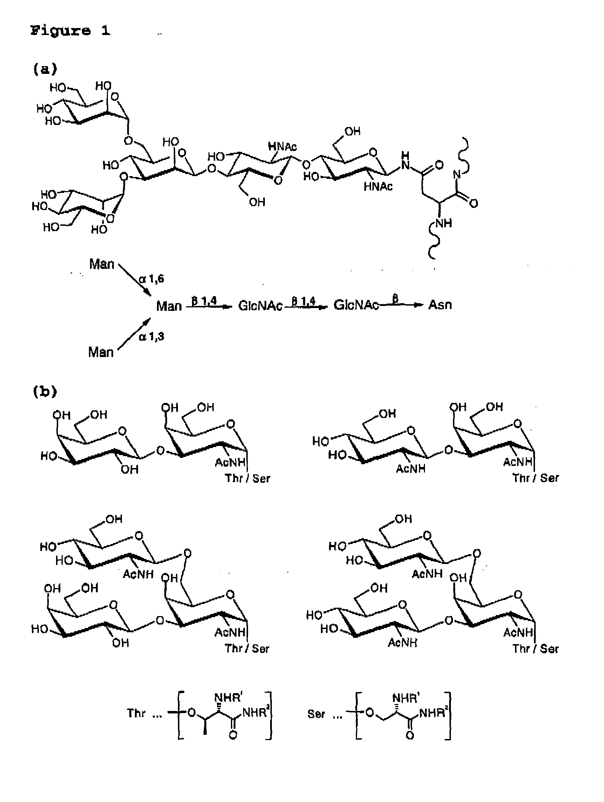

In the biosynthesis of N-glycosides one common saccharide precursor-unit (Glc₃Man₉GlcNAc₂), linked via a pyrophosphate bridge to dolichol, a long chain oligo isoprenol, hooked to the ER membrane, is added cotranslationally to polypeptides in the ER. The three glucosyl residues are removed by ER-resident α-glucosidases I and II, and the resulting MangGlcNAc₂ oligosaccharide is further processed in the ER and Golgi via pathways that are shared at least partially by all plant and animal cell types. Processing involves stepwise trimming by exoglycosidases and addition of new sugar residues catalyzed by glycosyltransferases. Thus all mammalian N-linked oligosaccharides share a common trimannosyl-chitobiose core, as shown in Figure 1(a), which is further processed to different types of structures. The biosynthesis of O-glycosylated glycoproteins is initiated in the Golgi by the addition of a single saccharide mainly to serine or threonine. In mammals the initiating sugar is normally α-N-acetylgalactosamine (GalNAc) although alternative pathways exist. There are at least seven O-oligosaccharide core structures. The four most common ones in mammalian glycoproteins are shown in Figure 1(b).

### Chemical glycosylation

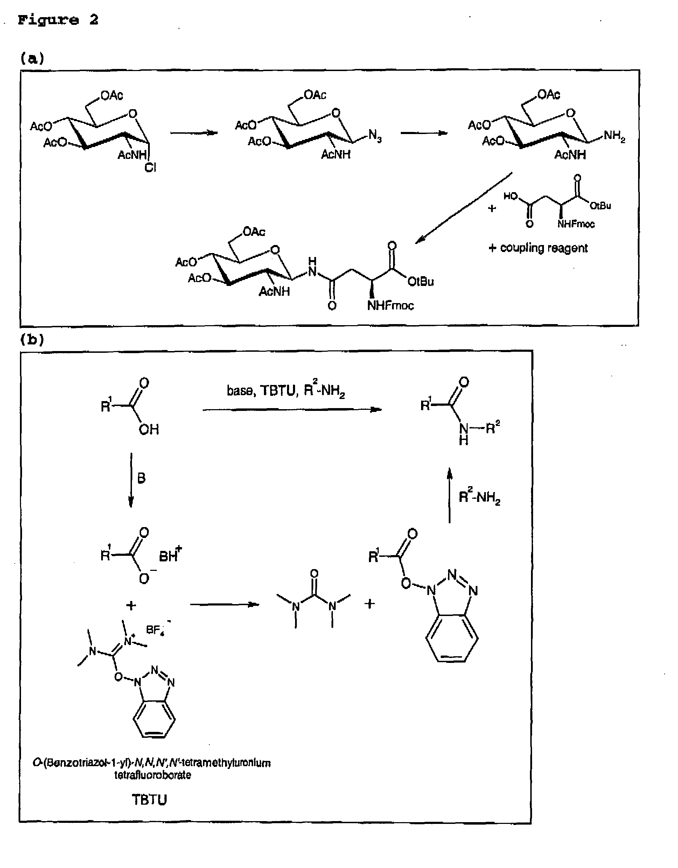

For chemical synthesis of glycopeptides and glycoproteins, the additional complexity and lability conferred by the attachment of the carbohydrate group to the protein or amino acid must be taken into account. A common approach is the synthesis of glycosyl amino acid building blocks, where the usually acetyl-protected sugar part is attached to one single protected amino acid, which is then subjected to common polypeptide synthesis, either in solution or on solid phase. Because of the base-sensitivity of 0-glycosidic linkages the final cleavage of the protective acetyl groups by Zemplén saponification have to be carefully adjusted, in order to avoid β-elimination, which is commonly observed. The usual procedures for the construction of N-glycosidic bonds involve peptide coupling between an aspartic acid derivative and a glycosylamine (see Figure 2(a)). The glycoslyamine can be synthesized by the treatment of glycosyl donors like halides with azide salts and subsequent reduction to the corresponding amine by hydrogenation, hydride reduction or Staudinger reaction. The coupling reactions of the prepared glycosylamines and protected aspartic acid derivatives are generally relying on the use of coupling reagents like TBTU or HBTU, originally developed for solid phase polypeptide synthesis. The reaction mechanism of those couplings using TBTU is shown in Figure 2(b). The chemical synthesis of O-glycosides is generally more complicated, due to the relatively labile O-glycosidic linkage. Again, glycosyl donors like glycosyl halides, thioglycosides or trichloroacetimidates are employed in the glycosylation of protected serine or threonine derivatives. Stereoselectivity concerning the newly formed glycosidic bond can be archived by the use of different promoters or methods already known from oligosaccharide synthesis. The obtained monosaccharide-amino acid building block can be used as acceptor for further glycosylation steps leading to O-glycosyl amino acids bearing complex carbohydrates. Unfortunately, very often several different protective steps have to be carried out in order to obtain certain structures. Direct glycosylation methods using fully elaborated oligosaccharidic glycosyl donors are also known, although they often show modest yields and poor selectivity, which can be tedious at an advanced stage of synthesis. Significant progress has also been made in the enzymatic oligosaccharide assembly of O-glycopeptides, where glycosyltransferases and endoglycosidases are used to build up oligosaccharidic structures from simple monosaccharidic glycopeptides. The synthesis of mature glycoproteins is then carried out by employing the previously synthesized glycosylated amino acid building blocks in solid phase polypeptide synthesis. Direct glycosylation procedures using polypeptide structures as glycosyl acceptors are rarely applied.

### Imino sugars and imino sugar conjugates

Imino sugars are sugar analoga with nitrogen in the ring instead of oxygen. Imino sugars are known as potent glycosidase inhibitors, since they show a strong interaction with the catalytic acid-base couple of the corresponding enzymes. There are important anti-viral, anti-cancer and diabetes type II medicaments based on iminosugars.

Alternatively to glycosidic-bond based glycoconjugates, iminosugar-amino acid conjugates may be used to derive new types of glycoconjugate mimetics and may be useful tools for biological studies. Iminosugar conjugates have not been found in nature so far, and only a few synthetic approaches were reported in the literature.

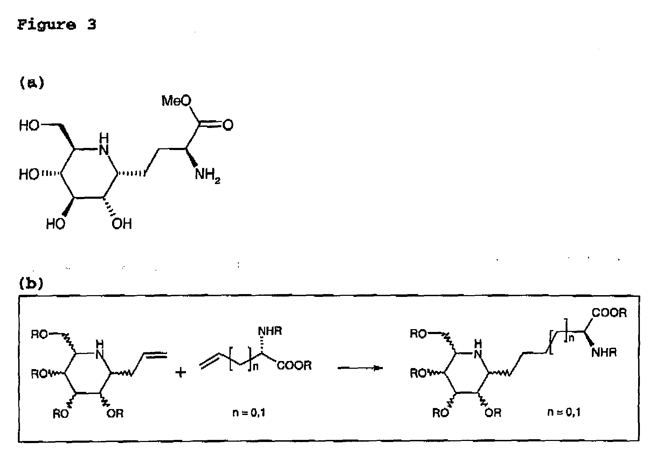

A first iminosugar-amino acid conjugate called nojirimycinyl C-L-serine (see Fig. 3), where the iminosugar moiety nojirimycin (the iminosugar analogon of D-glucose) is β-linked to an (S) -α-aminobutanoic acid residue serving as L-serine methylene isostere has been shown in T. Fuchss. R.R. Schmidt, Synthesis, 2000, 259. This molecule combines the presence of the ring nitrogen of iminosugars and the hydrolytically stable C-glycosidic linkage with the serine moiety and therefore is an interesting building block for generating new types of neoglycopeptides.

Very recently, an approach for the synthesis of various *C-*iminoglycosyl α-amino acids from *C*-allyl iminosugars via cross-metathesis (see Fig. 3(b)) has been reported (A. Dondoni et al., J. Org. Chem., 2005, 70, 5508). Using this type of reaction several C-iminoglycosyl α-aminopentanoates and α-aminohexanoates have been prepared.

The existing synthetic methods do not meet the needs of protein iminoglycosylation since non-naturally occurring amino acids like (S)-2-aminobut-3-enoic acid are used as synthetic precursors. Although iminosugar-amino acid building blocks can be prepared and used in oligopeptide synthesis, modification of existing oligo- or polypeptides composed of naturally occurring amino acids cannot be achieved.

### Summary of the invention

An object of the present invention is to provide a new class of iminosugar conjugates that do not show the above-mentioned drawbacks, and a method for the production of such iminosugar conjugates. Particularly the method according to the invention should allow the addition of iminosugar residues to existing peptides and protein backbones

These and other problems are solved by iminosugar conjugates according to independent claim 1, and a method for their production according to independent claim 4. Additional advantageous embodiments are described in the dependent claims.

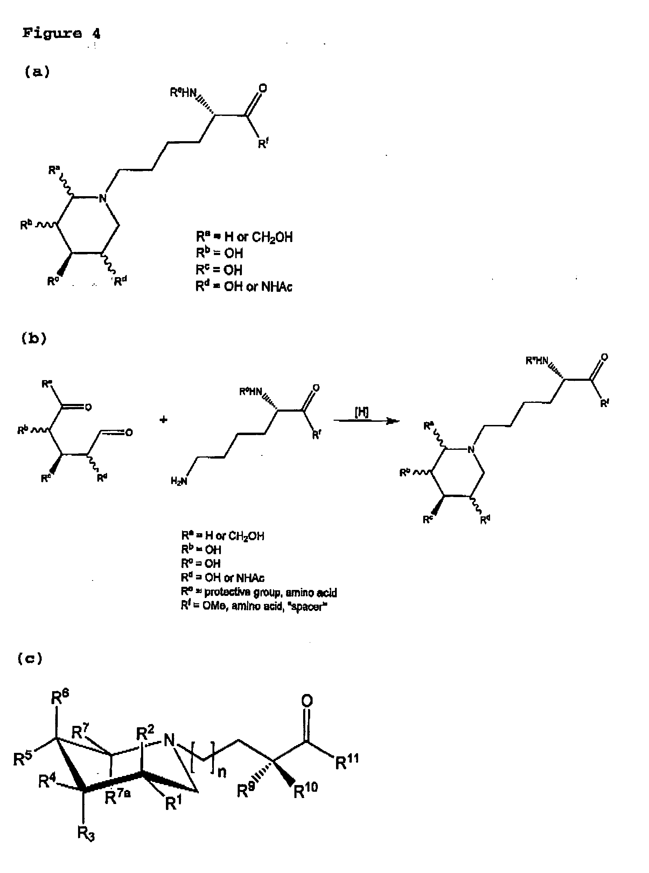

As can bee seen in the structure formula shown in Figure 4(a), the iminosugar-amino acid conjugates according to the invention are N-alkylated 1,5-dideoxy-1,5-iminohexitol or 1,5-dideoxy-1,5-iminopentitol derivatives.

The iminosugar component can be, for example, D-g*luco*-, L-*ido*-, D-*galacto*-, D-*manno*-, 2-acetamido-2-deoxy-D-*gluco*- or *xylo*-configuration. The N-substituent is a protected L-α-amino acid derivative, showing L-lysine-like structural features. The remarkable thing about these iminosugar-amino acid conjugates is the role of the ring nitrogen, which belongs to the iminosugar part, as well as to the amino acid, since it can be regarded as the substituted Nε-amino function of L-lysine. The linkage between the carbohydrate and the peptide component is not via the usual glycosidic position, but shows structural features of a very stable tertiary amine. Thus the linkage is very stable, also during Zemplén saponification conditions.

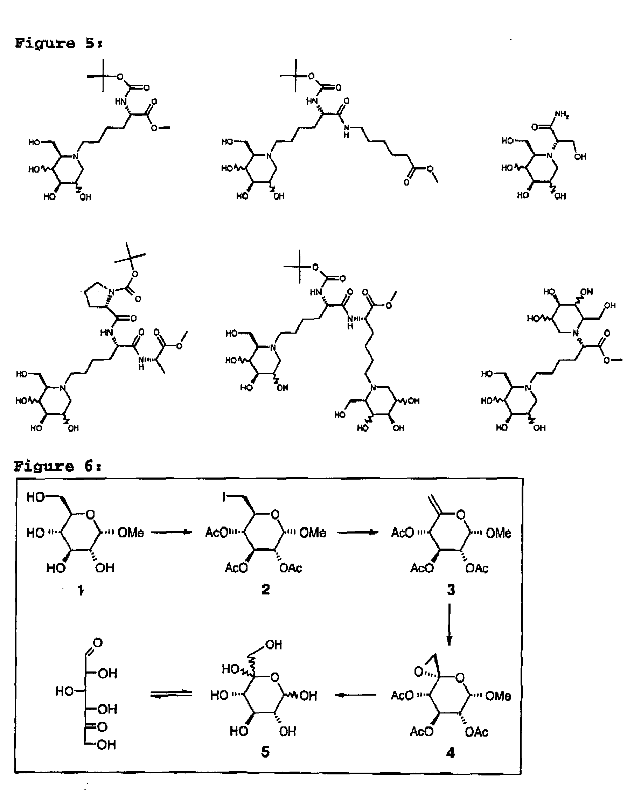

These new compounds are synthesised using -catalytic intramolecular reductive amination of dicarbonyl sugars with partially protected amino acids, as schematically shown in Figure 4(b). In the following this process shall be called "conjugation" for simplicity. The process of intramolecular reductive amination is carried out using Pearlman's catalyst (Pd(OH)₂/C) and H₂ at ambient pressure and room temperature. This heterogeneous catalytic reduction furnishes the desired D-configured iminosugars as main products, according to an empirical rule saying that the produced iminosugar moiety gets (R)-configuration at position C-5. Since unprotected ketoaldose derivatives can be used in the coupling step, no further deprotection is required. Additionally, lysine containing di- and tripeptides are used as amino acid components in order to prove the suitability of the method according to the invention for the "conjugation" of larger peptide fragments. Other amino acids, e.g. serine, are also employed as starting materials. In this case, iminosugar formation will occur at the N_{α}-amino functionality, leading to peptides bearing an iminosugar moiety at their N-terminus (see Figure 5).

A skilled person can straightforwardly apply the method for the preparation of iminosugar conjugates according to the invention to various types of educts. The resulting accessible class of iminosugar conjugate compounds is represented by the general structure shown in Figure 4(c).

The alkyl chain length parameter n can be freely chosen from n=0 upwards. Preferably n is between 0 and 10, and more preferably n is 2, 3, or 4. Residue R¹ can be chosen from H, OH, or NHAc, with Ac being Acetyl. R² can be H, OH, or NHAc R³, R⁴ R⁵, R⁶ can be H or OH. R⁷ and R⁸ can be H, CH₂OH, CH₃, COOH, or COOR with R being Alkyl or Aryl, R⁹ and R¹⁰ can be chosen from H, NH₂, NHR, with R being a protective group, an amino acid, a peptide, or a protein. R¹¹ can be OH, O-Alkyl, O-Aryl, NH₂, N-Alkyl, N-Aryl, amino acid or peptide, connected via an amide bond.

The resulting iminosugar-amino acid conjugates can be used as iminosugar building blocks in solid phase polypeptide chemistry to synthesize iminosugar containing glycopeptides and glycoproteins. In addition the corresponding reductive amination method according to the invention allows the production of iminoalditolated amino acids, peptides, and proteins, wherein lysine, the N-terminal end or other free amine groups may be modified, selectively and step by step.

This allows the synthesis of conjugates with all naturally occuring configurations in the carbohydrate moiety, the linkage between amino acids, peptides and proteins with oligosaccharides, and modifications with lipophilic and fluorophilic residues. The iminosugar moiety may be subject to further conjugation processes. Fluorescent or radionuclide containing groups, or groups specially suitable as mass spectrometry markers may be attached via the iminosugar moiety, which is interesting for analytical chemistry purposes.

### Detailed description of the invention

### Sugar educts

In order to synthesize the iminosugar conjugates according to the invention by intramolecular reductive amination as synthetic key step, 5-ketoaldose derivatives as sugar components have to be prepared. In general used synthetic approaches rely on the formation of 4- or 5-hexenopyranosides, combined with subsequent epoxidation and acid catalyzed hydrolysis to furnish the desired ketosugars. In the following the synthesis of some examples for sugar components is described.

### D-xylo-Hexos-5-ulose

In the first step of the presented synthesis of D-*xylo-*Hexos-5-ulose (see Figure 6), methyl α-D-glucopyranoside **1** is converted to the corresponding 6-deoxy-6-iodo compound, according to the procedure reported by P.J. Garegg et al., J. Chem. Soc., Perkin Trans I, 1980, 2866 (hereinafter referred to as Garegg method or Garegg's procedure). Using a heterogeneous reagent system consisting of PPh₃, I₂ and imidazole in dry toluene at 80-100 °C, a selective substitution of the primary hydroxyl function at position C-6 with iodine can be carried out in high yields without side reactions. Without further purification the water soluble reaction product can be subjected directly to a standard acetylation procedure, employing acetic anhydride in pyridine, to furnish the fully protected intermediate **2**. Elimination of HI with AgF in pyridine leads to enol ether **3** in high yields. The exocyclic double bond can be epoxidized using 3-chloroperbenzoic acid in CH₂Cl₂ in the presence of aqueous sodium bicarbonate. As reported in P.M. Enright et al., J. Org. Chem, 2002, 67, 3733, only the *(*5*R*)-stereoisomer of **4** is formed in this reaction process. The epoxide can be separated by flash column chromatography on a short plug of silica gel, although minor losses due to acid catalyzed hydrolysis cannot be avoided. These oxiranes are generally quite stable under slightly basic conditions. Therefore, Zemplén saponification using catalytic amounts of sodium methoxide can be carried out at -30°C without opening the oxirane. Finally, the addition of water and acidic ion exchange resin (Amberlite IR-120) results in spontaneous hydrolysis of the epoxide and the methyl acetal to furnish crude D-xylo-hexos-5-ulose **5.** TLC-analysis indicates the formation of a multicomponent mixture. Previous investigations have shown that ketoaldose **5** exists in at least seven different tautomeric forms in aqueous solution, due to its two anomeric centers at C-1 and C-5. Consequently, purification by silica gel chromatography is not possible and the crude reaction product has to be employed in the following reductive amination procedures.

### D-lyxo-Hexos-5-ulose

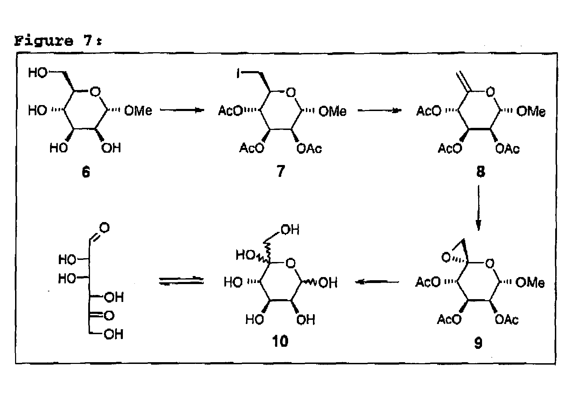

The synthesis of D-*lyxo*-hexos-5-ulose **10** (see Figure 7) relies on the same synthetic steps as the preparation of D-*xylo*-hexos-5-ulose **5**. In this case, methyl α-D-mannopyranoside **6** is used as starting material. After preparation of the corresponding peracetylated 6-deoxy-6-iodo compound **7,** enolether **8** and epoxide **9** can be prepared according to the procedures mentioned above. Again, the (5*R*)-stereoisomer of **9** is formed exclusively. Zemplén deacetylation under mild conditions and subsequent acid catalyzed hydrolysis furnish ketoaldose **10.** The crude product is processed without further purification, as it is composed of various tautomeric forms in aqueous solution.

### Methyl-(5RS)-2-acetamido-5-C-benzyloxy-2-deoxy-β-D-xylo-hexopyranoside

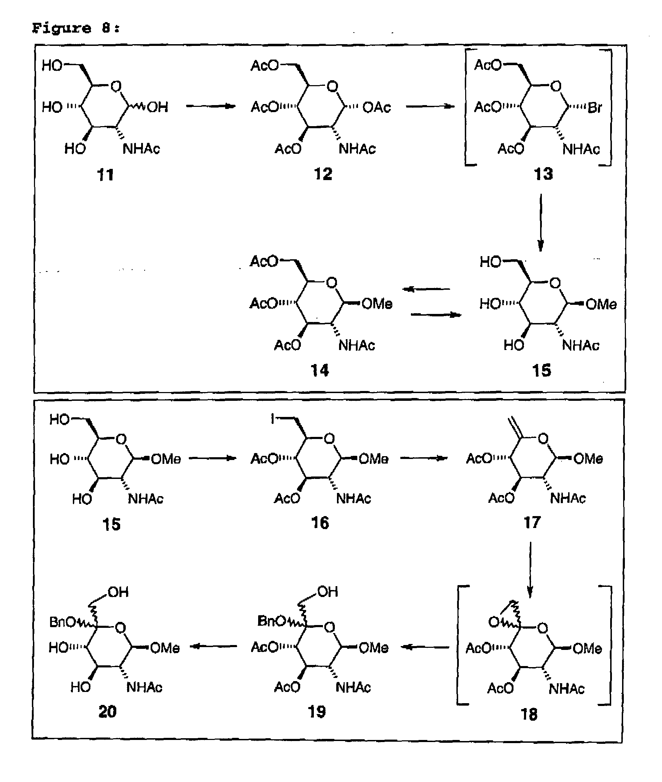

Figure 8 shows the synthesis starting from commercially available N-acetyl-n-glucosamine **11,** fully protected derivative **12** is synthesized by conventional peracetylation with acetic anhydride in dry pyridine. Treatment of **12** with a solution of HBr in acetic acid (5.7 M) leads to the unstable α-bromide **13,** which is immediately converted to methyl 2-acetamido-2-deoxy-β-D-glucopyranoside **15** by addition of 1.3 equivalents of sodium methoxide in dry methanol. Reaction of the β-bromide with methoxide causes the stereoselective formation of the β-configured glycosidic bond. Additionally, the acetyl protective groups are cleaved during this process. Since separation of the very polar reaction product from sodium bromide proves to be very difficult at this stage, crude **15** is peracetylated again to furnish compound **14,** which can be crystallized easily from ethyl acetate/cyclohexane. Zemplén saponification finally leads to pure **15.**

Treatment of methyl 2-acetamido-2-deoxy-β-D-glucopyranoside **15** with PPh₃, I₂ and imidazole in toluene-similar to Garegg's procedure - yields compound **16** which can be converted to the corresponding enol ether **17** using AgF in pyridine.

TLC-Experiments on the epoxidation of **17** with 3-chloroperbenzoic acid in CH₂Cl₂ show, that the formed oxirane **18** is too unstable to be processed in the next step. Immediate hydrolysis occurs on the TLC-plate, most likely resulting in the formation of covalent bonds to the active sites of the silica gel in the spotting area. Consequently, the epoxidation procedure is carried out in the presence of benzyl alcohol. Acid catalyzed regioselective opening of the oxirane intermediate takes place exclusively at C-5, as expected from theory. NMR experiments indicate the formation of both possible stereoisomers of structure **19** with the (5*S*)-configured one being the predominant species. Unfortunately, separation of the two products is not possible by column chromatography. Zemplén saponification finally yields the title compounds **20** in good yields. Parts of the (5*S*)-configured main product can be separated from the product mixture by chromatography and the configuration can be unambiguously assigned by NOESY NMR-experiments.

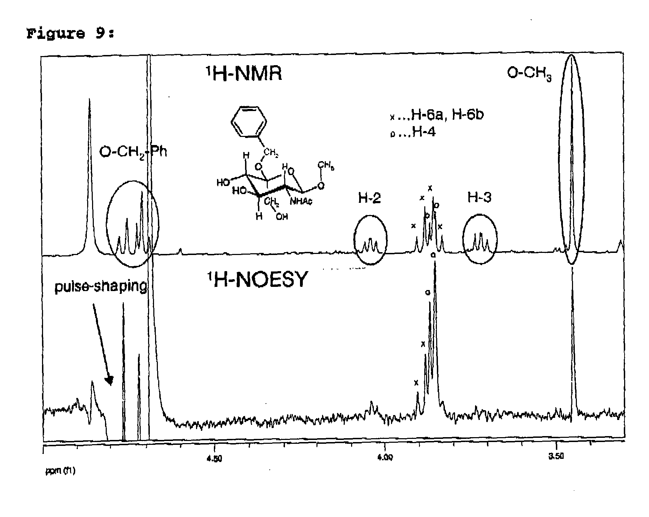

As the H1-NOESY and H1-NMR spectra in Figure 9 show, selective excitation of the protons of the benzyloxy function's CH₂-group shows NOEs to H-4 and H-2, as well as to the methoxy group in the anomeric position, indicating that the benzyloxy stubstituent is located *trans* to the functional groups at C-2 and C-4, and *cis* to the one at C-1. The mixture of diastereomers **20** can be directly taken into the next step of the synthesis. Reductive cleavage of the benzyl protective group during reductive amination and subsequent spontaneous hydrolysis of the resulting hemiacetal at C-5 finally lead to 2-acetamido-2-deoxy-D-xylo-hexos-5-ulose, regardless of the configuration of **20.**

### L-arabino-Hexos-5-ulose derivatives

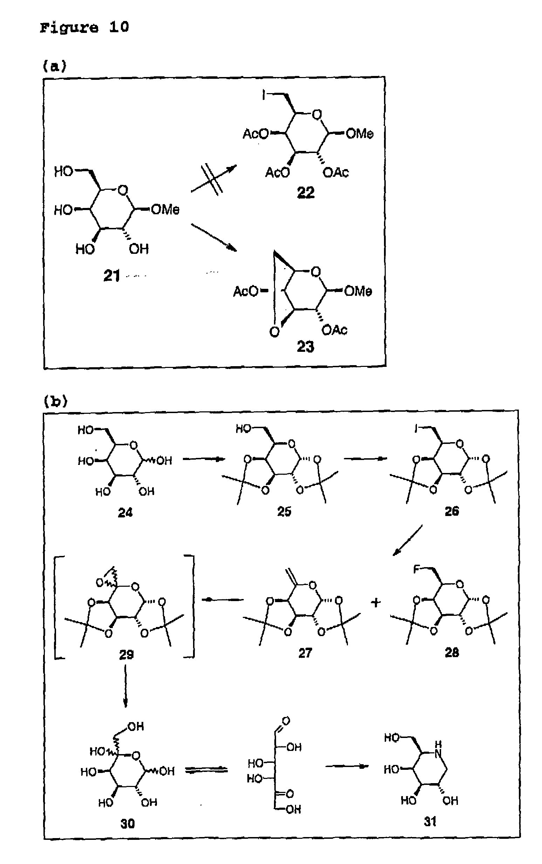

Since substitution reactions of the primary hydroxyl functions of unprotected methyl glycosides with iodine work very well in the previously reported cases, Garegg's procedure is applied to methyl β-D-galactopyranoside **21** (Figure 10(a)). Unfortunately, analysis after standard acetylation shows, that 3,6-anhydro compound **23** is the exclusive reaction product. Formation of the desired 6-deoxy-6-iodo-compound **22** is not observed. As the hydroxyl function at C-3 has proven to be too reactive for direct reaction of the unprotected methyl glycoside **21,** 1,2:3,4-di-O-isopropylidene-α-D-galactopyranose **25** is prepared from D-galactose **24** by acid catalyzed reaction with acetone (Figure 10(b)).

Now the corresponding 6-deoxy-6-iodo-compound **26** can be synthesized with the Garegg method. Elimination with silver(I)fluoride in pyridine proves to be very slow and more than 4 equivalents of this expensive reagent are required during a reaction time of 5 days. This synthetic step furnishes not only the desired enol ether **27,** but also the corresponding 6-deoxy-6-fluoxo-compound **28,** the latter in a yield of about 10% by conversion. Epoxidation of **27** with 3-chloroperbenzoic acid in CH₂Cl₂ in the presence of aqueous sodium bicarbonate furnishes the unstable oxirane **29,** which turns out to be too reactive to be purified by chromatographic means. Therefore, the crude product is treated with aqueous trifluoroacetic acid for deprotection and simultaneous epoxide hydrolysis. The resulting L-arabino-hexos-5-ulose **30,** akin to all the other previously described ketoaldoses, exists in various tautomeric forms. In order to establish the formation of the postulated reaction product, crude ketoaldose **30** is subjected to a reductive amination procedure using aqueous ammonia, catalytic amounts of Pearlman's catalyst and hydrogen at ambient pressure. The desired iminosugar 1-deoxygalactonojirimycin **31** can be isolated in an overall yield of 24% over the last three synthetic steps. The formation of the corresponding L-altro-configured iminosugar is not observed.

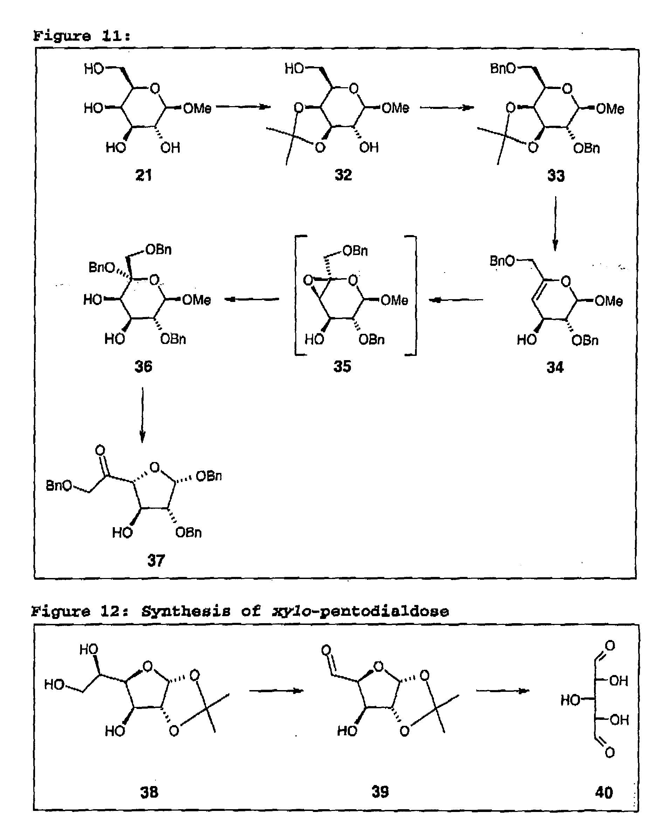

Although the synthesis of 1-deoxygalactonojirimycin **31** works out well, the handling of most of the synthetic intermediates proves to be quite difficult since the final preparative steps have to be carried out using crude products. Additionally, the formation of the hex-5-enopyranoside **27** tends to be quite time consuming and expensive, furnishing only moderate yields. Thus, a completely different, more promising, synthetic approach to L-arabino-hexos-5-ulose derivatives is outlined in the following (see Figure **11**). According to a synthesis of methyl (5R)-2,6-di-O-benzyl-5-C-benzyloxy-α-L-arabino-hexopyranoside **36,** described by Barili et al., Gazz. Chim. Ital., 1992, 122, 135, methyl β-D-galactopyranoside **21** is partially protected by acid catalyzed treatment with 2,2-dimethoxypropane to furnish **32.** After standard benzylation of the remaining unprotected hydroxyl functions, elimination of the C-4 substituent of product **33** under strongly basic conditions with t-BuOK in DMF yields the desired hex-4-enopyranoside **34.** Epoxidation with 3-chloroperbenzoic acid in CH₂Cl₂ in the presence of an excess of benzyl alcohol results in the formation of **36.** The intermediate epoxide **35** undergoes ring opening by acid catalyzed nucleophilic attack of the alcohol at C-5.

The L-arabino-hexos-5-ulose derivative **36** can be deprotected by catalytic hydrogenation to yield L-arabino-hexos-5-ulose **30.** Nevertheless, the benzyloxy group at C-5 proves to be very stable under these conditions. Thus, either low conversion rates or product losses due to reduction of the formed L-arabino-hexos-5-ulose **30** to L-tagatose have to be taken into account. This problem can be avoided by treatment of **36** with benzyl alcohol under strongly acidic conditions (pTSA•H2O, pH:1). This procedure furnishes benzyl 2,6-di-O-benzyl-β-L-arabino-hexofuranosid-5-ulose **37** as the only isolated product in nearly quantitative yield. Now compound **37** can be directly subjected to the reductive amination process according to the invention, revealing no major side products.

### xylo-Pentodialdose

In order to synthesize new *N*-substituted 1,5-dideoxy-1,5-iminoxylitol derivatives, xylo-pentodialdose **40** has to be prepared using, standard procedures (Figure 12). Although this sugar derivative is not a 5-ketoaldose but a dialdose, its synthesis is presented in this chapter in order to point out obvious structural analogies to the previously described compounds. Commercially available 1,2-0-isopropylidene-α-D-glucofuranose **38** is treated with sodium periodate in aqueous methanol at 0 °C until TLC shows complete conversion of the starting material. The crude protected dialdose **39** is subjected to acid catalyzed hydrolysis of the isopropylidene group in water. Since easily removable acidic ion-exchange resin (Amberlite IR-120) is used as catalyst, no further purification of the resulting xylo-pentodialdose **40** is necessary prior to the reductive amination process.

### Amino acid educts

The preparation of several amino acid derivatives is described, which can be used as educts in the synthesis of the iminosugar-aminoacid conjugates according to the invention. The amino acids have to be suitably protected to be adapted for the reductive amination process. Therefore, only those amino functions, that are supposed to react with the dicarbonyl sugars, remain unprotected. A different approach envisages the use of benzyloxycarbonyl protecting groups for those amino functions, since they are cleaved during catalytic reductive amination. The preparation of peptide derivatives for this purpose is outlined in the following.

### Amino Acid Derivatives

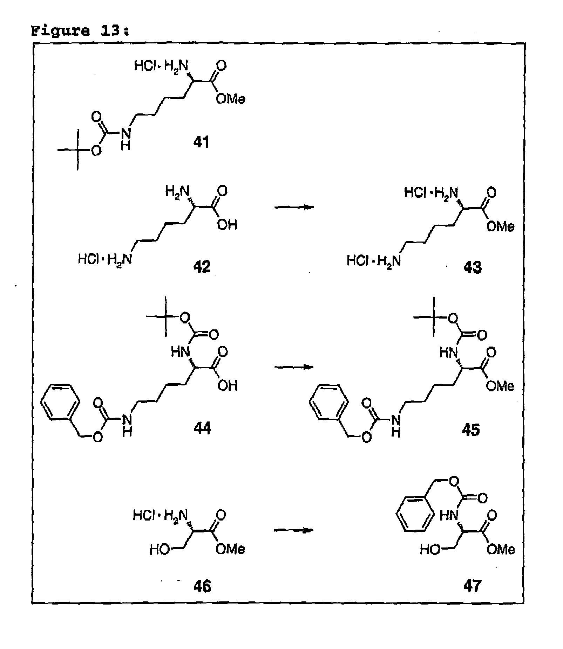

In order to synthesize iminosugar-amino acid conjugates that contain just one single amino acid residue, different L-lysine and L-serine derivatives have to be prepared, following standard procedures (see Figure 13). Commercially available H-L-Lys(Boc)-OMe hydrochloride 41 can be used directly for reductive amination of the N_{ε}-amino function. Selective reaction of the N_{ε}-amino functionality can be achieved by the use of Boc-L-Lys(Z)-OMe **45,** which can be prepared from Boc-L-Lys(Z)-OH **44** by simple treatment with TBTU and triethylamine in methanol at ambient temperature. For simultaneous reaction of both amino functions, L-lysine methyl ester dihydrochloride **43** can be used in the reductive amination process. Reaction of L-lysine hydrochloride **42** with SOCl₂ in dry methanol furnishes the corresponding methyl ester dihydrochloride **43** in high yield. Although commercially available L-serine methyl ester hydrochloride **46** could be directly employed, Z-L-Ser-OMe **47** is prepared by treatment of **46** with benzyl chloroformate in pure DMF in the presence of triethylamine. Compound **47** is very easy to handle in the reductive amination process, since the addition of base is not necessary to deprotonate the hydrochloride, as it would be necessary for **46.**

### Oligopeptide Derivatives

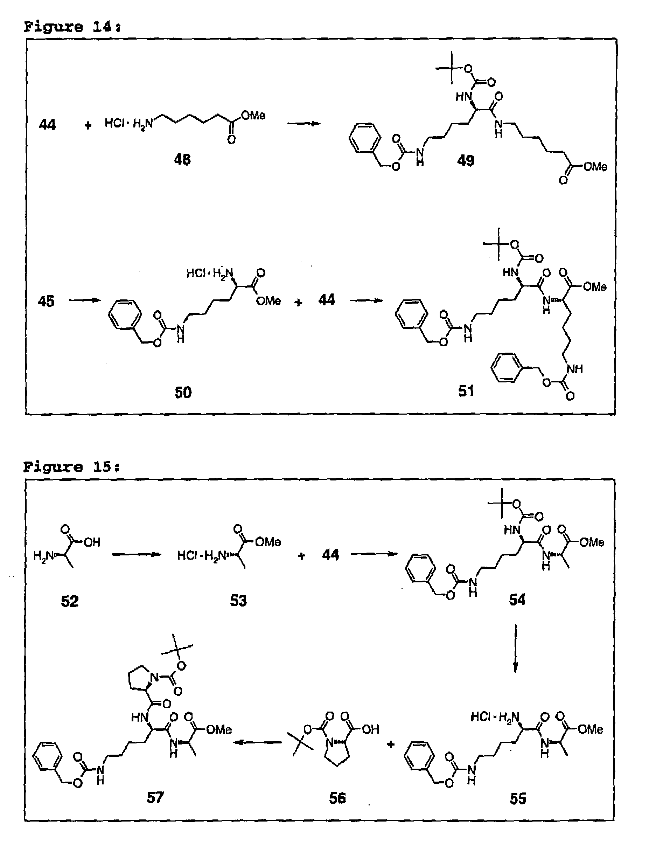

The preparation of L-lysine containing di- and tripeptide building blocks (see Figure 14) is carried out in solution, using standard peptide coupling procedures. Formation of an amide bond between BOC-L-Lys(Z)-OH **44** and methyl 6-aminocaproate hydrochloride **48** with TBTU in DMF in the presence of triethylamine furnishes compound **49** in quantitative yields. Boc-L-Lys(Z)-OMe **45** can easily be converted to H-L-Lys(Z)-OMe **50** by standard Boc-deprotection in dry methanol containing anhydrous HCl. The benzyl carbamate proves to be stable under these conditions. Peptide coupling of **50** with **44** using TBTU leads to Boc-L-Lys(Z)-L-Lys(z)-OMe **51**, a dipeptide suitable for catalytic reductive amination of both protected Ng-amino functionalities of the two lysine residues.

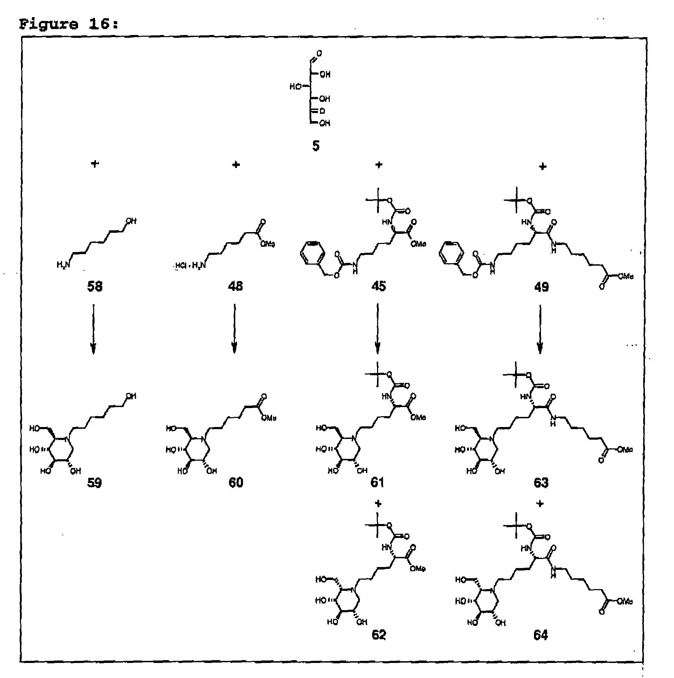

The preparation of tripeptide **57,** as shown in Figure 15, which resembles the C-terminal structure of the B-chain of pig-insulin, starts with the synthesis of L-alanine methyl ester hydrochloride **53** from L-alanine **52** by treatment with anhydrous HCl in methanol. Activation of **44** with TBTU and triethylamine enables peptide coupling with **53** to yield Boc-L-Lys(Z)-L-Ala-OMe **54.** After standard cleavage of the Boc-protective group as mentioned above, the coupling procedure can be applied to intermediate **55** and Boc-L-proline **56** to furnish the desired product **57** in high yields. Now Boc-L-Pro-L-Lys(Z)-L-Ala-OMe **57** can be subjected to catalytic reductive amination, reacting at the Z-protected amino function only.

### Catalytic Intramolecular Reductive Amination

The coupling procedure between the dicarbonyl-sugars and the amino acid derivatives by catalytic reductive amination according to the invention is now discussed in detail.

### 1-Deoxynojirimycin and 1-Deoxy-L-idonojirimycin Derivatives

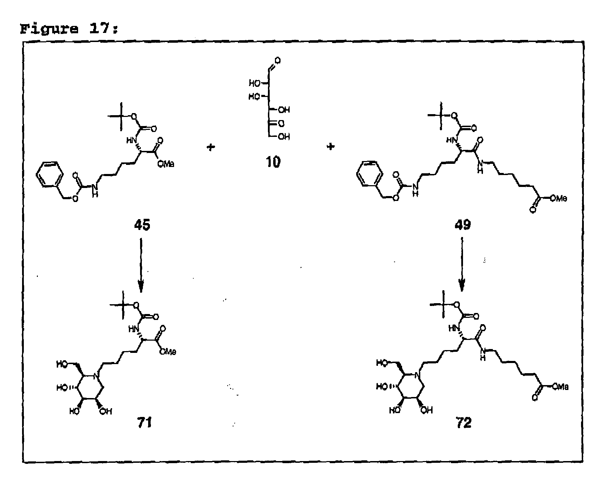

In order to show the suitability of the general synthetic concept of catalytic reductive amination for the preparation of iminosugar-aminoacid conjugates, crude *D-xylo*-hexos-5-ulose **5** is treated with 6-aminohexanol **58,** catalytic amounts of Pearlman's catalyst (Pd(OH)₂/C, 20%) and hydrogen at ambient pressure, using aqueous methanol as solvent (see Figure 16). After 60 hours reaction time, *N-*(6-hydroxyhexyl)-1-deoxynojirimycin **59** can be isolated by column chromatography from the reaction mixture in a yield of 61%. The formation of the corresponding L-*ido*-configured diastereomer is not observed. Reaction of **5** with 6-aminocaproate hydrochloride **48** under the same conditions, with additional use of triethylamine as basic agent, furnishes the desired D-*gluco*-configured product **60** in good yield as the only reaction product. Again, the reductive amination procedure proves to be stereoselective, nicely following to the empirical rule mentioned above.

As mentioned Perlman's catalyst (Pd(OH)₂ on activated carbon, e.g. 10 to 20%) can be used as a catalyst. According to further preferred embodiments of the present invention, the catalyst comprises Pd⁰ or Pt⁰ on activated carbon (e.g. 5 to 10%), or complex metal hydrides such as boranates and cyanoboranates. Raney-Nickel is a further suitable catalyst, however when using it, caution must be taken not to reduce the carbonyl group before it reacts with the amine group.

An alternative for the reductive amination step of the method according to the invention is the use of borohydrides, e.g. NaBH₃CN, Me₂NHBH₃, as soluble source of hydrogen.

Generally the method according to the invention can be carried out in methanol, water, or a mixture thereof. Of course other suitable solvents may also be used as long as sufficient solubility of the educts and products is given.

The reaction may be carried at atmospheric pressure or at higher pressures, preferably up to 100000 hPa.

Reductive amination of ketoaldose **5** and Boc-L-Lys(z)-OMe **45,** with concomitant cleavage of the Z-protecting group, furnishes the N-substituted 1,5-dideoxy-1,5-imino-D-glucitol **61** as well as the L-*ido* derivative **62** in a ratio of about 4:1. Although the R_{f} values of the reaction products are very similar, the obtained mixture of diastereomers can be separated by repeated column chromatography on silica gel. Moreover, reaction of 5 with compound **49** also results in a mixture of diastereomeric compounds **63** and **64.**

### 1-Deoxymannonojirimycin Derivatives

In analogy to D-xylo-hexos-5-ulose **5**, crude D-lyxo-hexos-5-ulose **10** is used as sugar component in the reductive amination procedure according to the invention (Figure 17), using Pearlman's catalyst and hydrogen in aqueous methanol at ambient pressure and temperature. Reaction of **10** with the amino acid building blocks **45** and **49** furnishes the desired D-manno-configured products **71** and **72,** respectively, in fair overall yields. In both cases the formation of the corresponding L-*gulo*-isomers is not observed.

### 2-Acetamido-1,2-dideoxynojirimycin Derivatives

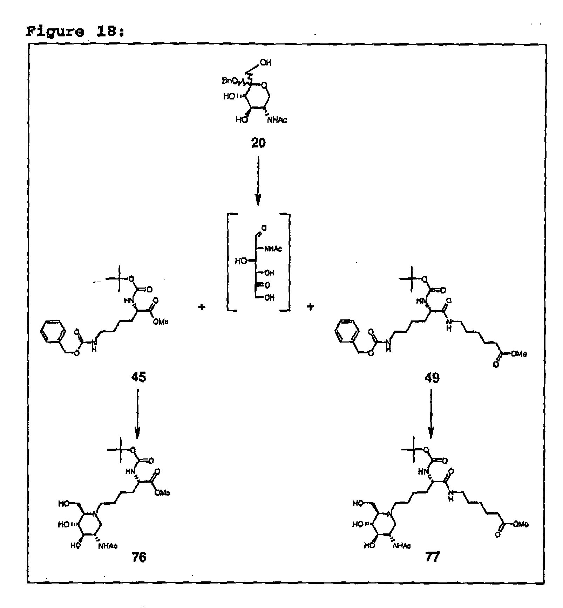

For the synthesis of 2-acetamido-1,2-dideoxynojirimycin derivatives (Figure 18), the diastereomeric mixture of methyl (5*RS*)-2-acetamido-5-*C*-benzyloxy-2-deoxy-o-D-*xylo-*hexopyranosides 20 described above is employed as starting material. Reaction of 20 with hydrogen in aqueous methanol in the presence of Pearlman's catalyst at ambient pressure results in the formation of the intermediate 2-acetamido-2-deoxy-D-xylo-hexos-5-ulose due to cleavage of the benzyl protecting group and spontaneous hydrolysis of the resulting hemiacetals. After one hour, quantitative conversion of the starting material can be observed by TLC-analysis. The Z-protected amino acid derivatives **45** and **49,** respectively, are added to the reaction mixture and the reaction is continued under the same conditions for about 60 hours. Z-Deprotection and subsequent intramolecular reductive amination furnish the desired 2-acetamido-1,2-dideoxy-D-*gluco*- target compounds **76** and **77,** which are isolated in yields of 60-70 % by column chromatography on silica gel. The formation of L-*ido*-configured side products is not observed in these cases.

### 1-Deoxygalactonojirimycin Derivatives

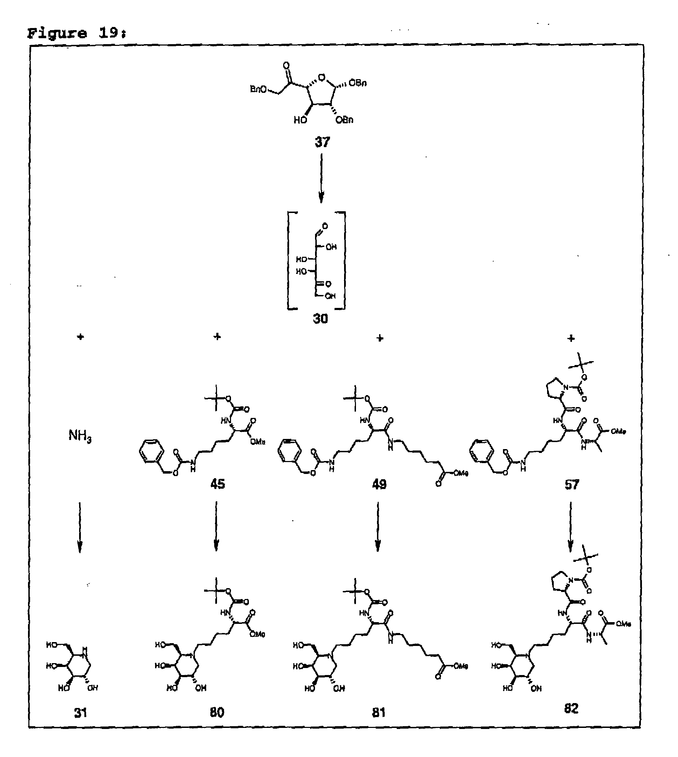

Benzyl 2,6-di-O-benzyl-β-L-amino-hexofuranosid-5-ulose **37** is employed as starting material for the synthesis of 1-deoxygalactonojirimycin derivatives (see Figure 19). Treatment of **37** with Pearlman's catalyst and hydrogen in methanol at ambient pressure and temperature for one hour results in the formation of the corresponding intermediate L-arabino-hexos-5-ulose **30.** The complete conversion of the starting material is indicated by TLC-analysis. Various amines or *N*-Z-protected amino acid components can be added and continued reaction results in reductive amination of ketoaldose **30** to the corresponding iminosugar derivatives. When aqueous ammonia is added to **30,** 1-deoxygalactonojirimycin **31** can be isolated as the only product after **40** hours in 72% yield, proving the suitability of the applied starting material for catalytic intramolecular reductive amination. Reaction of **30** with **45** and **49** furnishes the iminosugar-amino acid conjugates **80** and **81** in 70 and 55% yield, respectively. In order to attempt "conjugation" of larger peptide components, tripeptide **57** is employed in the same manner, resulting in the formation of **82.** In all cases, no L-altro-configured side products can be isolated or identified by NMR-analysis. H-L-Lys(soc)-OMe hydrochloride **41** is used as amino acid component to perform reductive amination of the α-amino function of lysine (Figure 20). Reaction with **30** in the presence of triethylamine under standard conditions leads to a product mixture of **86** and **87** in a ratio of about 7:3, which cannot be separated by column chromatography on silica gel. In order to obtain one single reaction product, the mixture of **86** and **87** is treated with concentrated aqueous ammonia at room temperature for two hours, resulting in quantitative conversion to the corresponding amide **90.** Further purification of **90** is not necessary.

### 1,5-Dideoxy-1,5-iminoxylitol Derivatives

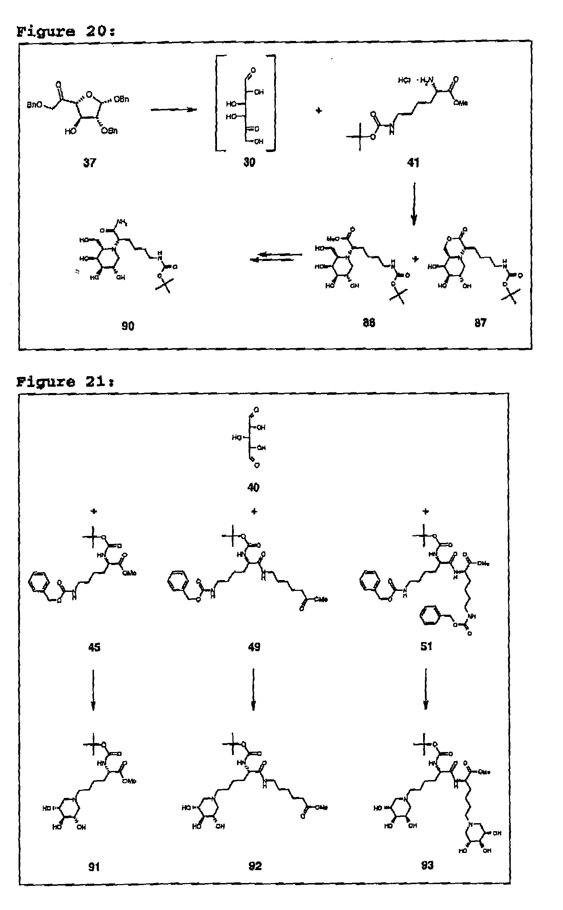

For the synthesis of 1,5-dideoxy-1,5-iminoxylitol derivatives, easily available *xyl*o-pentodialdose **40** is used as sugar component, as shown in Figure 21. Reductive amination of crude **40** with **45** and **49,** respectively, in aqueous methanol in the presence of Pearlman's catalyst and hydrogen at ambient pressure and temperature furnishes the desired products **91** and **92** in yields of about 70%. Similar reaction of **40** with dilysine derivative **51** results in the formation of target compound **93** in high yield. Since xylo-pentodialdose **40** shows two aldehyde functionalities, the reductive amination procedure proceeds very fast, resulting in a shortening of the reaction time needed for complete conversion. Additionally, the achieved overall yields are higher than in the previously reported cases. The obtained iminosugars are meso-configured, showing a mirror plane as an element of symmetry.

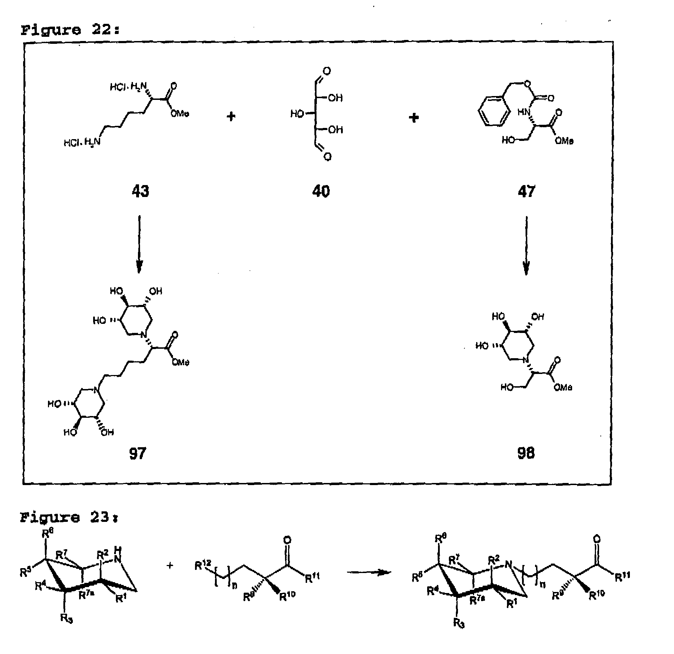

Further reductive amination reactions according to the invention are shown in Figure 22. Reductive amination of **40** with L-lysine methyl ester dihydrochloride **43** in the presence of triethylamine yields L-lysine derivative **97.** This compound features two iminosugar moieties, formed by reaction of both available amino functions of **43** with two equivalents of *xylo*-pentodiasdose **40.** In order to prove the suitability of the applied method for the "conjugation" of amino acids other than lysine, Z-L-Ser-OMe **47** is used as amino acid component. Reductive amination with **40** furnishes compound **98** in 73% yield, indicating that reductive amination of the α-amino function of various amino acids with dicarbonyl sugars is generally possible.

An alternative method to produce iminosugar conjugates according to a further preferable embodiment of the invention is the nucleophilic substitution reaction as shown in Figure 23. Starting from the iminosugar. R¹ is chosen from H, OH, or NHAc; R² is chosen from H, OH, or NHAc; R³, R⁴, R⁵, and R⁶ are chosen from H or OH; R⁷ and R^{7a} are chosen from H, CH₂OH, CH₃, COOH, COO-Alkyl, or COO-Aryl, whereby at least R⁷ or R^{7a} is chosen to be H.

In the second educt R⁹ and R¹⁰ is chosen from H, NH₂, NHR, with R being a protective group, an amino acid, a peptide, or a protein; R¹¹ is chosen from OH, O-Alkyl, O-Aryl, NH₂, N-Alkyl, N-Aryl, amino acid or peptide, connected via an amide bond; and R¹² is chosen from Br, Cl, I, Tosyl, Brosyl, Nosyl, Mesyl, and other residues suitable to react with an amine group in a nucleophilic substitution reaction.

### List of figures

- Figure 1(a) :: Structure of the common mammalian trimannosylchitobiose core of *N*-glycosylated glycopeptides.
- Figure 1(b) :: Four most common mammalian O-oligosaccharide core structures.
- Figure 2(a) :: Synthesis of *N*-glycosyl amino acid building blocks.
- Figure 2(b) :: Amide formation using TBTU as coupling reagent.
- Figure 3(a) :: Nojirimycinyl *C*-L-serine derivative.
- Figure 3(b) :: Synthetic route to various *C*-iminoglycosyl α -amino acid derivatives.
- Figure 4(a) :: Iminosugar conjugate according to the invention.
- Figure 4(b) :: Synthetic approach via the intramolecular reductive amination process according to the invention.
- Figure 4(c) :: Generalised structural formula of Iminosugar conjugate according to the invention.
- Figure 5:: Examples of iminosugar conjugates according to the invention.
- Figure 6:: Synthesis of D-*xylo*-hexos-5-ulose.
- Figure 7:: Synthesis of D-*lyxo*-hexos-5-ulose.
- Figure 8:: Synthesis of methyl (5*RS*)-2-acetamido-5-*C*-benzyloxy-2-deoxy-β-D-xylo-hexopyranoside.
- Figure 9:: H1-NOESY and H1-NMR spectra of (5S)-2-acetamido-5-C-benzyloxy-2-deoxy-β-D-*xylo*-hexopyranosade.
- Figure 10(a):: Outcome of Garegg's procedure applied to methyl β-D-gasactopyranoside.
- Figure 10(b):: Synthesis of L-*arabino*-hexos-5-ulose and 1-deoxygalactonojirimycin.
- Figure 11:: Synthesis of benzyl 2,6-di-O-benzyl-β-L-*arabino*-hexofuranosid-5-ulose.
- Figure 12:: Synthesis of *xylo*-pentodialdose
- Figure 13:: Amino acid derivatives.
- Figure 14:: Dipeptide derivatives.
- Figure 15:: Synthesized tripeptide derivative.
- Figure 16:: Reductive amination of D-*xylo*-hexos-5-ulose.
- Figure 17:: Reductive amination of D-*lyxo*-hexos-5-ulose.
- Figure 18:: Reductive amination of 2-acetamido-2-deoxy-D-*xylo*-hexos-5-ulose.
- Figure 19:: Reductive amination of L-*arabino*-hexos-5-ulose.
- Figure 20:: Reductive amination of L-*lysine's* α-amino function.
- Figure 21:: Reductive amination of *xylo*-pentodialdose.
- Figure 22:: Further reactions of *xylo*-pentodialdose.
- Figure 23:: Alternative synthesis of iminosugar conjugates according to the invention.

## Claims

1. Compound with structural formula 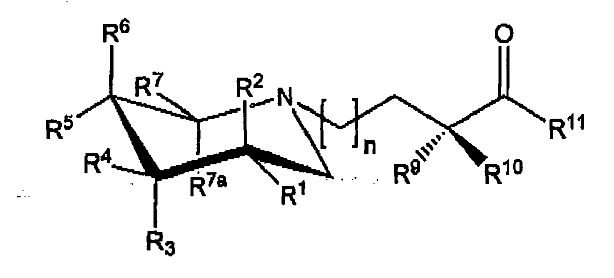 with integer n≥0; R¹, R², being chosen from H, OH, NHAc, OGlycosyl, O(CH₂)ₘCH₃, or O(CF₂)ₘCF₃; R³, R⁴, R⁵, and R⁶ being chosen from H, OH, OGlycosyl, O(CH₂)ₘCH₃, or O(CF₂)ₘCF₃; with integer m between 1 and 10; R⁷ and R^{7a} being chosen from H, CH₂OH, CH₃, COOH, COO-Alkyl, or COO-Aryl, whereby at least R⁷ or R^{7a} is chosen to be H; R⁹ and R¹⁰ being chosen from H, NH₂, NHR, with R being a protective group, an amino acid, a peptide, or a protein; and R¹¹ being chosen from OH, O-Alkyl, O-Aryl, NH₂, N-Alkyl, N-Aryl, amino acid or peptide, connected via an amide bond.

2. Compound according to claim 1, **characterized in that** n lies between 0 and 10.

3. Compound according to claim 1, **characterized in that** n is 2, 3, or 4.

4. Method for producing a compound according to any of the above mentioned claims, wherein a first compound with structural formula 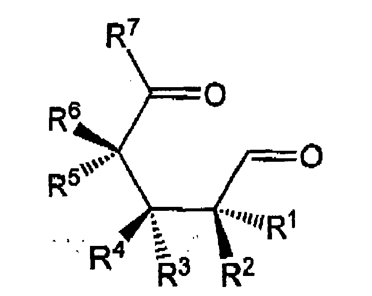 with R¹, R², being chosen from H, OH, NHAc, OGlycosyl, 0(CH₂)ₘCH₃, or O(CF₂)ₘCF₃; R³, R⁴, R⁵, and R⁶ being chosen from H, OH, OGlycosyl, O(CH₂)ₘCH₃, or O(CF₂)ₘCF₃; with integer m between 1 and 10; R⁷ being chosen from H, CH₂OH, CH₃, COOH, COO-Alkyl, or COO-Aryl;
is reacted with a second compound with structural formula 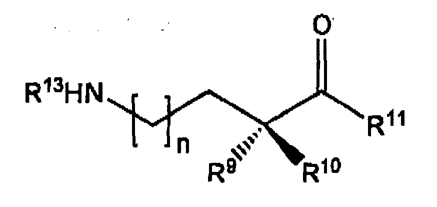 with R⁹ and R¹⁰ being chosen from H, NH₂, NHR, with R being a protective group, an amino acid, a peptide, or a protein; R¹¹ being chosen from OH, O-Alkyl, O-Aryl, NH₂, N-Alkyl, N-Aryl, amino acid or peptide, connected via an amide bond, and R¹³ being H or a protective group removable by hydrogenolysis.

5. Method according to claim 4, **characterised in that** the reaction between the first and second compound is carried out with H₂ and a catalyst chosen from the group consisting of Pearlman's catalyst and Pd or Pt on activated carbon.

6. Method according to claim 4, **characterised in that** the reaction between the first and second compound is carried out with a borohydride in solution.

7. Method according to any of claims 4 to 6, **characterised in that** the reaction is carried out at atmospheric or higher pressure up to 100000 hPa.

8. Method according to any of claims 4 to 7, **characterised in that** the reaction is carried out at room temperature.

9. Method according to any of claims 4 to 8, **characterised in that** the reaction is carried with a solvent comprising methanol and/or water.

10. Method for producing a compound according to any of the above mentioned claims, wherein a first compound with structural formula 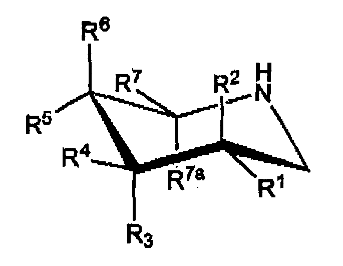 with R¹, R², being chosen from H, OH, NHAc, OGlycosyl, O(CH₂)ₘCH₃ , or O(CF₂)ₘCF₃; R³, R⁴, R⁵, and R⁶ being chosen from H, OH, OGlycosyl, O(CH₂)ₘCH₃, or O(CF₂)ₘCF₃; with integer m between 1 and 10; R⁷ and R^{7a} being chosen from H, CH₂OH, CH₃, COOH, COO-Alkyl, or COO-Aryl, whereby at least R⁷ or R^{7a} is chosen to be H;
is reacted with a second compound with structural formula 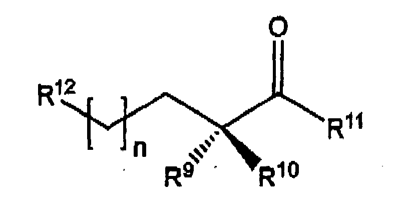 with R⁹ and R¹⁰ being chosen from H, NH₂, NHR, with R being a protective group, an amino acid, a peptide, or a protein; R¹¹ being chosen from OH, O-Alkyl, O-Aryl, NH₂, N-Alkyl, N-Aryl, amino acid or peptide, connected via an amide bond; and R¹² being chosen from Br, Cl, I, Tosyl, Brosyl, Nosyl, Mesyl, and other residues suitable to be replaced by the amine group of the first compound in a nucleophilic substitution reaction.

11. Peptide compound, modified with a iminosugar moiety added to a free amine group of the peptide with a method according to any of claims 4 to 9.

12. Protein compound, modified with a iminosugar moiety added to a free amine group of the peptide with a method according to any of claims 4 to 9.
